# EUROPEAN PATENT SPECIFICATION

(11) **EP 3 395 189 B1**
(45) Date of publication and mention of the grant of the patent: **11.08.2021**
(21) Application number: 16879137.4
(22) Date of filing: 11.10.2016
(51) Int. Cl.: A23N 17/00, B01F 13/00, B01F 15/00, B65G 21/20, B65G 15/00, F24F 3/16, F24F 7/007, A23J 1/14, A23J 3/16

(54) **CONTINUOUS FERMENTATION DEVICE**
KONTINUIERLICHE FERMENTATIONSVORRICHTUNG
DISPOSITIF DE FERMENTATION CONTINUE

(30) Priority: 24.12.2015 KR 20150186520
(43) Date of publication of application: 31.10.2018
(73) Proprietor: CJ Cheiljedang Corporation, Seoul 04560 (KR)
(72) Inventor: HONG, Young Ho, Suwon-si Gyeonggi-do 16708 (KR); LEE, Hwa Jeong, Suwon-si Gyeonggi-do 16509 (KR); KIM, Hong Ki, Incheon 21949 (KR); ROH, Won Bae, Incheon 21376 (KR); JUNG, Sang Kwan, Incheon 21941 (KR); HAN, Sang Ik, Incheon 21969 (KR); PARK, Seung Won, Yongin-si Gyeonggi-do 16894 (KR); CHO, Seong Jun, Seoul 04971 (KR)
(74) Representative: Frei Patent Attorneys
(86) International application number: PCT/KR2016/011379
(87) International publication number: WO 2017/111269

(56) References cited:
- CN-B- 101 717 716
- CN-B- 102 304 557
- KR-B1- 100 322 481
- KR-B1- 100 587 732
- KR-B1- 100 689 671
- KR-Y1- 200 206 287
- KR-Y1- 200 257 316
- KR-Y1- 200 257 316
- US-A- 4 559 235

## Description

### [Technical Field]

The present disclosure relates to a fermentation device, and more specifically, to a continuous fermentation device which can perform fermentation of continuously fed objects to be fermented while transporting the objects.

### [Background Art]

As a source of vegetable proteins for livestock, raw grain materials or by-products which remain after the extraction of active ingredients from the raw grain materials are mainly utilized as raw materials for feed. Typical examples of such vegetable proteins are soybean meal, corn gluten, *etc.* Soybean meal is a dehulled and defatted component of soybean meal and is currently used as various kinds of feeds of livestock, such as pigs, chickens, beef cattle, *etc.* Currently, soybean meal has problems in that it has a lower protein content compared to animal proteins, insufficient contents of some vitamins, minerals, and unknown growth factors (UGF), and also contains various kinds of anti-nutritional factors (ANFs), thus decreasing digestibility when used as feed. In particular, since anti-nutritional factors such as trypsin inhibitor can decrease digestibility in young livestock and thereby inhibit their growth, the amount of soybean meal to be added to feed has been limited.

To solve the above problems, studies have been performed for the purpose of decreasing the content of anti-nutritional factors while improving digestibility through fermentation using microorganisms, and soybean meal with improved quality is currently used for various livestock feeds both home and abroad. A representative example is soybean meal.

A representative method for preparing fermented soybean meal is as follows. A beneficial microorganism is introduced into soybean meal and the soybean meal is stored in a batch fermenter for a certain period of time. The soybean meal fermented by the beneficial microorganism is dried and crushed, and used as a main source of vegetable proteins to be added in the compound feed.

The main production facilities for preparing the fermented soybean meal may be largely classified into a high-temperature steam facility capable of lowering the population of general microorganisms contaminating soybean meal, a solid fermentation facility for performing solid fermentation by mixing soybean meal with a beneficial microorganism, and a drying facility for drying the fermented soybean meal after fermentation, and a crushing facility for crushing the fermented soybean meal to a size to be used as feed. The most important part requiring technological advance in the process of preparing the fermented soybean meal is the part of the solid fermentation facility. Based on the presumption that beneficial microorganisms will have the same effect and the same fermentation time, it is speculated that the effect and efficiency of the solid fermentation equipment in the manufacturing facility of the fermented soybean meal can realize a high quality product and reduction of manufacturing costs.

Basically, the solid fermenter for fermented soybean meal may vary depending on the kind of the beneficial microorganism. In a case where an aerobic microorganism is used, temperature and air permeability may be considered as major factors. For effective control of temperature and ventilation, temperature is mostly controlled within the batch fermenter.

The batch fermenter as disclosed in KR 2015-0008265 A below is mainly used due to its excellent fermentability and simple operation technique. However, in the case of mass production in the feed industry, the requirement of the number of fermenters and the size of the fermenters are a source of major investment costs.

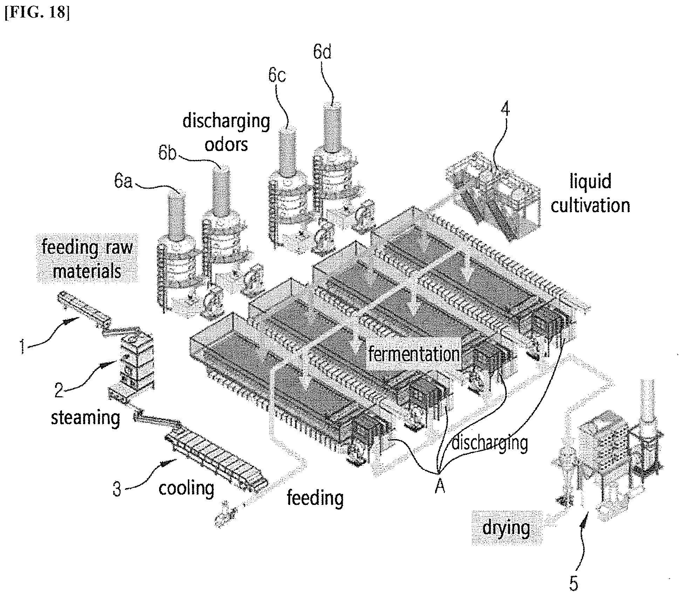

As shown in FIG. 18, the conventional batch fermentation device performs fermentation as follows. Raw materials (objects to be fermented) fed into the input unit (1) are distributed and fed into a plurality of fermentation modules (A) by undergoing steaming and cooling processes in steaming unit (2) and cooling unit (3), and the seed microorganism cultured in the cultivation unit (4) is introduced into a plurality of fermentation modules (A) to ferment the objects to be fermented. Additionally, a plurality of odor-processing units (6a, 6b, 6c, and 6d) for treating odors generated during the fermentation process must be provided in each fermentation device (A), and the completed objects to be fermented are sucked through the suction unit (5) connected to each fermentation device (A) through a pipe and transferred to a drying unit. However, the conventional batch fermentation device has disadvantages in that, due to the characteristics of the batch fermentation, cleaning time for removing contaminants in the fermentation device, time for feeding the strain and the raw materials, and time for discharging the fermentation product are required; the pipes may be clogged during the suction of the fermented objects to be fermented; and additionally, the productivity of the final product per fermenter is very low because each of these processes is intermittently performed in each fermentation device (A).

Accordingly, there is a need for the development of a fermenter required for solid fermentation of soybean meal and other grains in a cost-effective manner. Additionally, there is a need for the development of a continuous fermentation device so as to improve the productivity of the batch fermenter.

### [Disclosure]

### [Technical Problem]

Under these circumstances, the present disclosure is contrived based on the conventional problems described above so as to solve the problems thereof. Accordingly, an object of the present disclosure is to provide a continuous fermentation device which enables continuous fermentation of objects to be fermented so that feeding of a raw material, performing fermentation, and discharging of a fermentation product, *etc.* in one facility, thereby preventing problems that may occur during the operation owing to the simplified process and facility and reducing the costs for constituting the facility by reducing manufacturing facilities.

CN 102 304 557 B discloses a fermenter in which air supplied from a blower fan is supplied through an air outlet tube which is connected to the inside of a conveyor belt constituting fermentation beds.

From KR 200 257 316 Y1, a fermenter is known in which air holes for supplying air to objects to be fermented is located at the lowest part of a plurality of conveyor belts, thus supplying air from the bottom part to the top part of a conveyor belts

### [Technical Solution]

To achieve the above object, the present disclosure provides a continuous fermentation device including a plurality of fermentation modules, each fermentation module including:
a conveyor belt, which transports objects to be fermented that are continuously fed thereinto from one side to the opposite side thereof;
agitators, which are located on the conveyor belt; and
air diffusers, which are located on one side of the conveyor belt,
wherein the continuously fed objects to be fermented are fermented while being passed through the fermentation modules and are continuously discharged, according to claim 1.

The plurality of fermentation modules are preferably in a stacked form where one side and the opposite side of each conveyor belt are connected to each other, and the continuously fed objects to be fermented are continuously fermented while being passed through the plurality of fermentation modules.

The plurality of conveyor belts of the plurality of fermentation modules preferably form a stack of two rows, and each of the plurality of fermentation modules further includes a belt connection part, which connects one side and the opposite side of each of the conveyor belts in each row, and the continuously fed objects to be fermented are continuously fermented while being passed through the fermentation modules.

Each of the agitators preferably includes:
a shaft, which is located on the conveyor belt;
a plurality of support frames, wherein each support frame radially extends from one side and the opposite side of the outer circumference of the shaft;
connecting frames, wherein each connecting frame connects the ends of the support frames located at each of the opposite sides of the shaft; and
a plurality of agitating frames, wherein each agitating frame connects the connecting frames on one side and the opposite side.

Each of the agitators preferably agitates the objects to be fermented, which are transported on the conveyor belt as each of the agitating frames moves, as the shaft rotates.

The continuous fermentation device preferably includes agitation wings, in which each agitation wing is located on one side of the outer circumference of the plurality of agitating frames in the longitudinal direction.

The shaft is preferably height-adjustable.

The air diffuser includes:
a main supply pipe, which is located across the top side of the conveyor belt on one side of the conveyor belt; and
distribution pipes, wherein each distribution pipe extends from one side of the main supply pipe and is located in the longitudinal direction of the top side of the conveyor belt, and a plurality of apertures are formed around the periphery thereof.

The apertures are preferably formed in at least two rows and the objects to be fermented are supplied with air through the apertures.

Each of the distribution pipes injects air inside the objects to be fermented when the objects to be fermented are transported on the conveyor belt.

Each row formed by the apertures is preferably arranged at a predetermined angle from the center of the distribution pipes.

The apertures are preferably arranged in three rows on one side of the outer circumference of the distribution pipes (320), in which each row formed by the apertures is arranged with an angular difference of from 60 degrees or greater to 90 degrees or less from the center of the distribution pipes.

The continuous fermentation device preferably further includes guides, which have a predetermined height and are located in the longitudinal direction on both sides of the conveyor belt.

The continuous fermentation device preferably further includes a conveyor belt which transports the objects to be fermented that are continuously fermented and discharged to a drying unit.

The continuous fermentation device preferably further includes:
a plurality of air conditioning devices, which are provided on one side of each of the conveyor belt for suction of odors generated during the fermentation process of the objects to be fermented; and
odor-processing units, which are connected to the plurality of air conditioning devices,
in which the odors sucked from the plurality of air conditioning devices are integrated and processed in the odor-processing units.

### [Advantageous Effects of the Invention]

As described above, the continuous fermentation device according to the present disclosure enables continuous performance of feeding of a raw material, performing fermentation, discharging of a fermentation product, *etc.* in one facility, and as a result, the processing time can be significantly reduced, thereby reducing the operation time of the production process. Accordingly, the continuous fermentation device of the present disclosure can improve business competitiveness by reducing investment costs, improving productivity, *etc.* in the development of mass production through solid fermentation, thereby having the effect of significantly reducing the manufacturing costs of products manufactured therefrom.

### [Brief Description of Drawings]

FIG. 1 is an overall perspective view of a continuous fermentation device according to an embodiment of the present disclosure.
FIG. 2 is a side view of a continuous fermentation device according to an embodiment of the present disclosure.
FIG. 3 is an overall perspective view of a continuous fermentation device according to another embodiment of the present disclosure.
FIG. 4 is a side view of a continuous fermentation device according to another embodiment of the present disclosure.
FIG. 5 is a perspective view illustrating the configuration of an agitator in detail.
FIG. 6 is a perspective view illustrating the configuration of an air diffuser in detail.
FIGS. 7A and 7B are cross-sectional views taken along the line A-A' in FIG. 6, illustrating the arrangement of the apertures and the air injection angles.
FIG. 8 is a plan view of the conveyor belt portion viewed from above.
FIG. 9 shows one side of a cross-section taken along the line B-B' in FIG. 8.
FIGS. 10 to 13 show the comparison results with respect to fermentation effects between the conventional batch fermentation device and a continuous fermentation device according to the present disclosure: a graph on the number of live microorganisms (FIG. 10), a graph on water content (FIG. 11), a graph on crude proteins (CP) (FIG. 12), and SDS-PAGE results (FIG. 13), respectively.
FIGS. 14 to 16 show the comparison results with respect to fermentation effects between the conventional batch fermentation device and a continuous fermentation device according to the present disclosure: a graph on the number of live microorganisms (FIG. 14), a graph on a graph on crude proteins (CP) (FIG. 15), and water content (FIG. 16), respectively.
FIG. 17 shows an overall fermentation process of objects to be fermented using the continuous fermentation device according to the present disclosure.
FIG. 18 shows a fermentation process of objects to be fermented according to a conventional batch fermentation device.

### [Best Mode]

The above objects, features, and other advantages of the present disclosure will become more apparent by describing in detail preferred embodiments of the present disclosure with reference to the accompanying drawings. During this process, the thicknesses of the lines and the sizes of the components shown in the drawings may be exaggerated for clarity and convenience of explanation. Additionally, the following terms are defined in consideration of the functions in the present disclosure, which may vary depending on the intentions or customs of the user or operator. Therefore, the definitions of these terms must be described based on the contents throughout this specification.

Furthermore, the embodiments described are provided for illustrative purposes of explanation and are not intended to limit the technical scope of the present disclosure.

Each component of the continuous fermentation device of the present disclosure may be used as an integral unit or as an independent unit, as needed. In addition, some components of the continuous fermentation device may be omitted depending on the forms of usage.

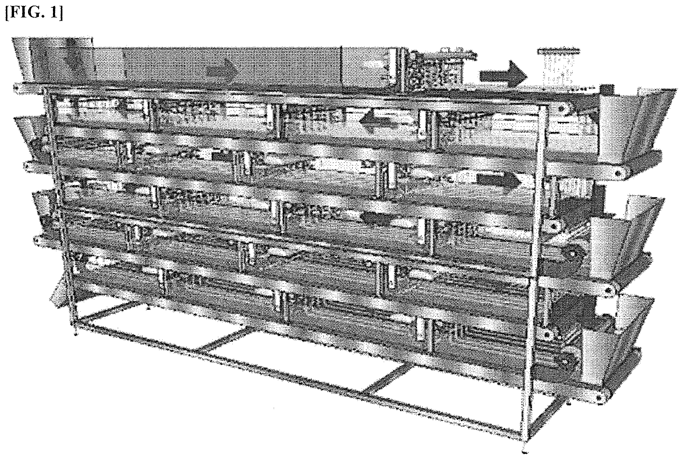

Hereinafter, a continuous fermentation device according to an embodiment of the present disclosure will be described in detail with reference to accompanying FIGS. 1 to 17.

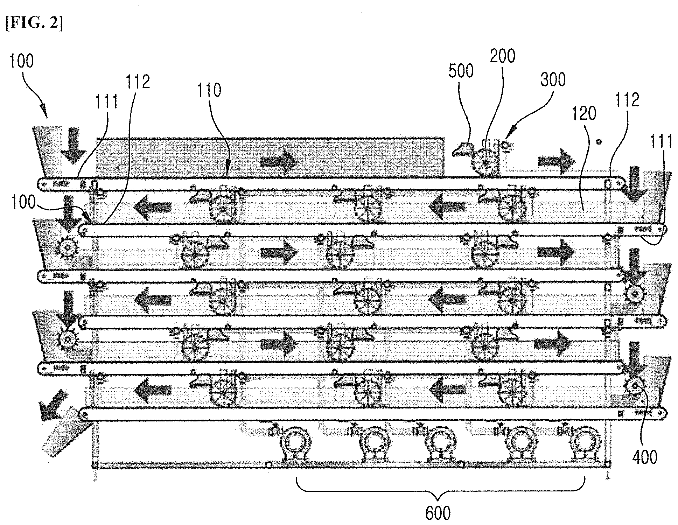

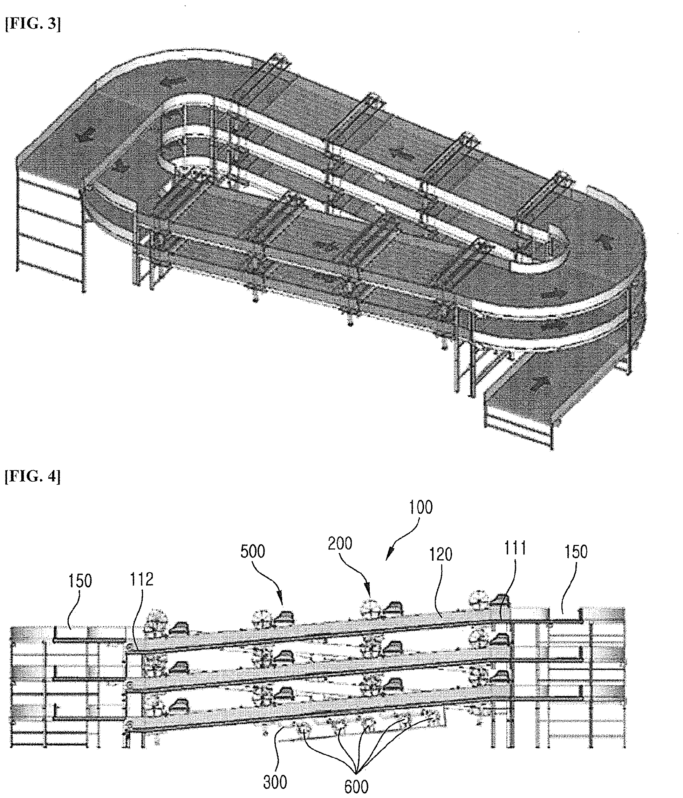

As shown in FIGS. 2 and 4, the continuous fermentation device of the present disclosure may include fermentation modules (100), where each fermentation modules (100) is provided with a conveyor belt (110), agitators (200), air diffusers (300), and guides (120); and as further shown in FIGS. 1 and 3, the continuous fermentation device of the present disclosure may be configured in a form where a plurality of fermentation modules (100) are stacked, whereby the installation space can be efficiently utilized.

The conveyor belt (110) is located in a predetermined longitudinal direction so that objects to be fermented arriving at one side (111) of the conveyor belt (110) are transported to be discharged through the opposite side (112).

The agitators (200) are located on the conveyor belt (110) and agitate the objects to be fermented which are transported by the conveyor belt (110).

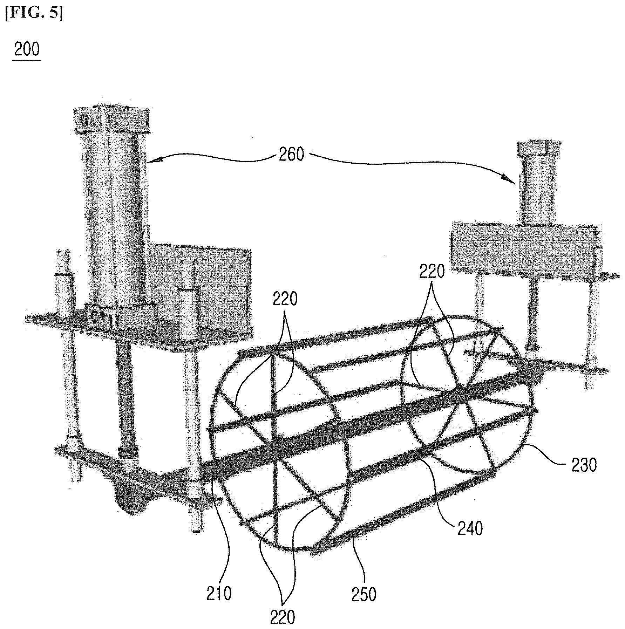

Describing the configuration of the agitators (200) in more detail by referring to FIG. 5, the agitators (200) may include a shaft (210), support frames (220), connecting frames (230), agitating frames (240), and cylinders (260).

The shaft (210) is located being spaced a predetermined distance upward across the transport direction of the conveyor belt (110).

Each of the plurality of support frames (220) radially extends from one side and the other side of the shaft (210), and each connecting frame (230) connects each end of the support frames (220).

Each agitating frame (240) is located across each connecting frame (230) to act upon rotation of the shaft (210), and the objects to be fermented that are transported on the conveyor belt (110) are agitated by the agitating frames (240).

The agitating frames (240) may be provided with an agitation wing (250) located along the longitudinal direction thereof, and as such, the area of contact with the objects to be fermented can be increased, thereby enabling a more effective agitation.

Additionally, the cylinders (260) connected to both sides of the shaft (210) allow the up- and-down movement of the shaft, and thereby, the agitation height can be adjusted according to the height of the objects to be fermented that are transported.

When the temperature of the objects to be fermented rises above the appropriate fermentation temperature by the heat generated during the fermentation, the agitators (200) agitate the objects to be fermented and thereby lower the temperature of the objects so that an appropriate temperature can be maintained.

Each air diffuser (300) is located on one side of the conveyor belt (110) and supplies air to the objects to be fermented.

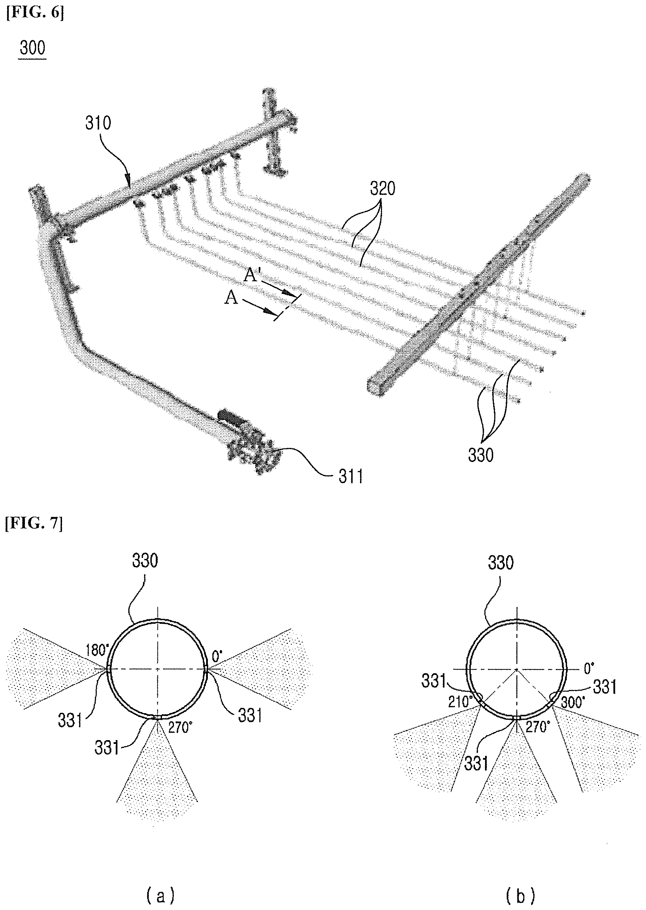

Explaining the configuration of each air diffuser (300) in more detail by referring to FIG. 6, each air diffuser (300) includes a main supply pipe (310) and distribution pipes (320).

The main supply pipe (310), being extended on the conveyor belt (110), is located across the conveyor belt (110), and air is supplied thereto through an air supply unit (600) connected to one side thereof. The amount of air being supplied from the air supply unit (600) into the main supply pipe (310) can be controlled using an air-controlling means such as a valve (311), *etc.,* disposed on the main supply pipe (310).

Each distribution pipe (320) is connected to the main supply pipe (310) and extended toward the transport direction of conveyor belt (110) so as to have a certain length. The distribution pipe (320) may be provided in a plurality according to the width of the conveyor belt (110), and a plurality of apertures (331) are formed around the periphery thereof so that the air introduced through the main supply pipe (310) can be distributed through a plurality of distribution pipes (320) and supplied through the apertures (331) to the objects to be fermented being transported on the conveyor belt (110).

Specifically, the objects to be fermented are stacked up to a certain height and transported on the conveyor, and as the objects to be fermented are transported, each distribution pipe (320) becomes disposed inside of the stacked objects to be fermented, thereby enabling oxygen supply into the inside of the stacked objects to be fermented.

Additionally, the plurality of apertures (331) may be disposed to be formed in two or more rows. Furthermore, referring to FIGS. 7A and 7B, effective ventilation to the objects to be fermented can be achieved by pre-setting the intervals between the rows formed by the apertures (331) and the angle of the air being injected from the apertures (331) before air injection, according to the amount of the objects to be fermented. In addition, as shown in FIG. 7, when the apertures (331) of the distribution pipe (320) are formed in three rows, the intervals between the rows of the apertures (331) may be arranged to be formed at 90 degrees (FIG. 7A) or 60 degrees (FIG. 7B), respectively, from one side of the distribution pipe (320). This is because, in the fermentation of a solid material such as soybean, unlike in a liquid state, the air supply may encounter resistance in a solid state, and thus, the number of the apertures (331) and the intervals between the apertures are important for uniform air delivery to the objects to be fermented. That is, when the apertures (331) are formed in only one row and the pressure for the air to be supplied is significantly increased, the air may be delivered to the entirety of the objects to be fermented. However, in this case, the objects to be fermented located near the apertures will be over-supplied with air, which causes drying rather than fermentation, thus making it difficult to achieve uniform fermentation over the entirety of the objects to be fermented. Accordingly, uniform fermentation can be achieved by supplying air to the entirety of the objects to be fermented by disposing the apertures (331) in two or more rows so that the intervals therebetween are arranged at a predetermined angle from the center.

Additionally, each distribution pipe (320) at one end portion thereof is provided with a cap capable of opening and closing of the distribution pipe (320), and thus, the inside of each distribution pipe (320) can be cleaned by easily opening and closing the cap.

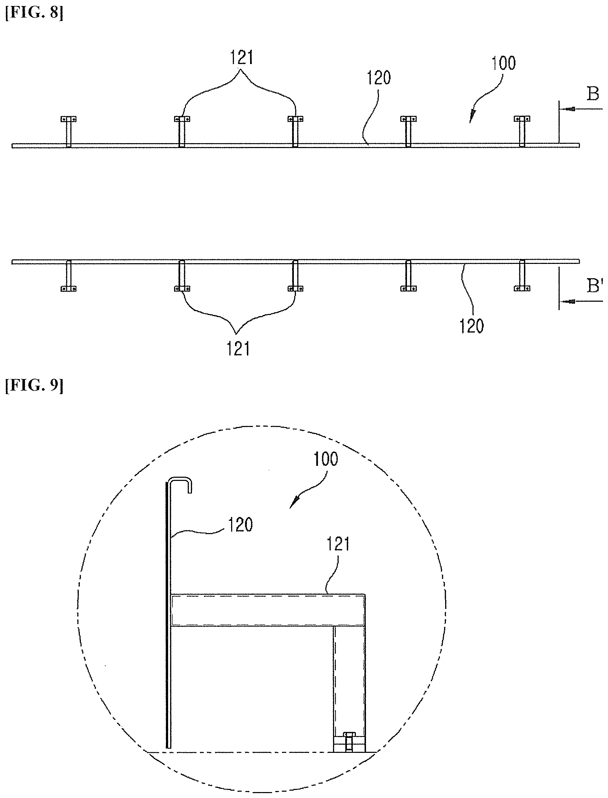

Guides (120) are located in the longitudinal direction on both sides of the conveyor belt (110), and referring further to FIG. 8, the guides (120) have a predetermined height on both sides of the conveyor belt (110), and supporting units (121) that support the guides (120) may be located at one side of each of the guides (120).

That is, the objects to be fermented transported on the conveyor belt (110) are transported while being passed through between the guides (120). When the objects to be fermented are transported on the conveyor belt (110), a phenomenon occurs in which the objects to be fermented are spread on both sides. The falling-off of these objects to be fermented from both sides of the conveyor belt (110) may be prevented by allowing extra space on both sides. However, in such case, the width of the conveyor belt (110) will become greater, thus lowering utilization of the space. Meanwhile, by providing the guide (120) on both sides of the conveyor belt (110), the spreading phenomenon of the objects to be fermented on the conveyor belt (110) and the resulting falling-off of the objects can be prevented and the width of the conveyor belt can also be minimized.

Additionally, on the top side of each conveyor belt (110), a plurality of air conditioning devices (500) for removing odors generated during fermentation of the objects to be fermented are installed. Each of the air conditioning devices (500) is connected to a single odor-processing unit (60), and thus there is an advantage in that the odors collected by suction in each air conditioning device (500) can be treated in the single odor-processing unit (60).

In an embodiment, a case where a plurality of fermentation modules (100) are stacked as described above is explained as follows, again referring to FIGS. 1 and 2. A plurality of fermentation modules (100) are stacked in a row and one side (111) and the opposite side (112) of each fermentation module (100), specifically, one side (111) and the other side (112) of each conveyor belt (110) are disposed in an alternating manner. That is, the fermentation is performed in such a manner that the objects to be fermented fed into one side (111) of the conveyor belt (110) are transported into the opposite side (112) and discharged, and the discharged objects to be fermented are again transported from one side (111) of the conveyor belt (110) located on a lower part thereof to the opposite side (112) thereof (see arrows in FIGS. 1 and 2).

Additionally, a hopper that guides the objects to be fermented, which are discharged from a conveyor belt (110) and fed into the conveyor belt (110) on a lower location thereof, and a pulverizing unit (400) which pulverizes the objects to be fermented may be located between each of the fermentation modules (100). Additionally, the air diffusers (300) are connected to each other and disposed as such, and air can be supplied from the air supply unit (600) located furthest to the bottom to the inter-connected air diffusers (300).

Additionally, in another embodiment, a case is explained where a plurality of fermentation modules (100) are stacked as described above, focusing on the difference compared to the above embodiment as follows, again referring to FIGS. 3 and 4. A plurality of fermentation modules (100) are stacked in two rows, and sides (111) and the opposite sides (112) of fermentation modules (100) located on different rows from each other are disposed to be connected by a belt connection part (150). The shape of the belt connection part (150) is not limited, but it may be in a curved shape for smooth transport of the objects to be fermented. Accordingly, the fed objects to be fermented are sequentially transported from a conveyor belt (110) in one row to a conveyor belt (110) in another row through the belt connection part (150) in an approximately elliptical shape, and thereby the objects to be fermented are fermented (see arrows in FIGS. 3 and 4).

Additionally, the conveyor belt (110') is located connecting the opposite side of the fermentation module (100) in the final position to a drying unit, and the fermentation products, which are continuously discharged upon completion of fermentation, are transported to the drying unit (not shown).

Accordingly, the continuous fermentation device of the present disclosure can effectively transport and dry a fermentation product being continuously discharged, and enables smooth transport and drying of the fermentation product without clogging of pipes, *etc.,* unlike the conventional batch fermentation device utilizing a suction method.

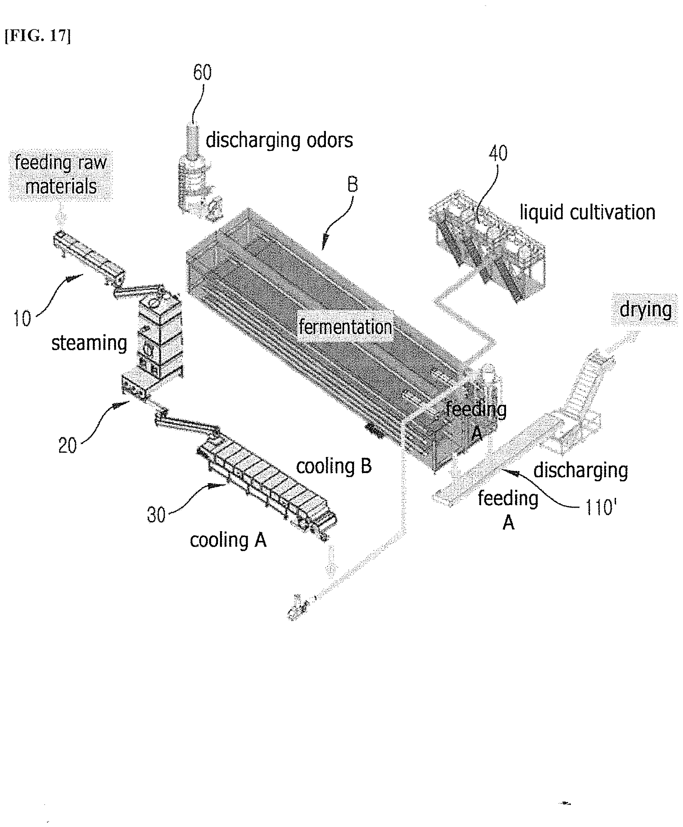

Hereinafter, again referring to FIGS. 1 to 17, the continuous fermentation device according to the present disclosure is described as an experimental example to explain the difference from the conventional batch fermentation device. To confirm the differences in fermentability and quality with the existing batch fermentation device, solid fermentation was performed by applying the continuous fermentation device according to the present disclosure. As shown in FIG. 17, the raw material *(i.e.,* the objects to be fermented) introduced through the feeding unit 10, was fed into a fermentation device (B) consisting of a plurality of fermentation modules (100) after undergoing steaming and cooling processes in steaming unit (20) and cooling unit (30), the seed microorganism cultured in a cultivation unit (40) was added thereto and the objects to be fermented were continuously fermented, and the fermentation product being discharged upon completion of fermentation was transported to a drying unit through the conveyor belt (110') and dried.

For this experiment, soybean meal was used as the raw material (*i.e.,* the objects to be fermented) and the amount of soybean meal used was 60 kg to 90 kg based on the raw material. For the effective fermentation of the raw material (*i.e.,* soybean meal), water was added (water content: at a 43% level), and then, steaming was performed to sterilize the soybean meal to which the water was added. During the steaming process, the average temperature of the soybean meal was maintained at 70°C to 90°C for 20 minutes. After the steaming process, the soybean meal underwent a cooling process and the seed microorganism was inoculated so as to perform the solid fermentation. As the seed microorganism, *Plantarum K2G,* a subspecies of *Bacillus amyloliquefaciens,* was used. For the stable growth of the seed microorganism, the seed microorganism was inoculated at a concentration of 10% relative to the weight of the raw material so as to secure the total number of the microorganism of 1×10⁷ cfu/mL or greater at the initial stage. The cultivation of the seed microorganism was activated in GYP medium (glucose 10.0 g, yeast extract 8.0 g, and polypeptone 2.0 g (pH 7.0)).

The objects to be fermented were fed to a height of 20 cm as a standard and samples were collected at 4-hour intervals for confirming the growth process of the seed microorganism and the change in quality of the fermentation product. The changes in the viable cell count (cfu/g), water content (%) of the fermentation product, and crude protein content (%) of each sample were measured.

For the replacement of the conventional batch fermentation device, it is extremely important that the same quality of the fermentation product under the same conditions be ensured. In the conventional batch fermentation, the fermentation of a seed microorganism is completed within 24 hours and the quality (viable cell count: 1×10⁹ cfu/g or greater, final water content: 32% or less, and increase of crude protein: 6% or greater). As such, to achieve the above quality (improvement of an increase rate of the crude protein), the control of the temperature of the fermentation product and air permeability for oxygen supply were optimized through the agitators (200) and air diffusers (300). That is, to further facilitate the permeability of oxygen to the objects to be fermented as described above, the distribution pipes (320) of the air diffusers (300) were inserted into the objects to be fermented, whereby the air permeability can be improved and the change in the overall quality of the fermentation product can be minimized.

After fermentation, the temperature of the objects to be fermented was controlled as follows while observing the change in temperature of the objects to be fermented. When the temperature of the fermentation product was increased to a certain temperature (45°C) or higher, the agitators (200) were operated, and the temperature of the objects to be fermented was induced to decrease through the contact of the objects to be fermented with air by these agitators (200), and thereby the temperature change of the objects to be fermented from 47°C to 39°C was confirmed. In addition, the temperature change by the agitators (200) alone was confirmed and the effect of air diffusion during the agitation was confirmed.

As shown in Table 1 below, when the agitators (200) were operated alone, the temperature change before and after the agitation was at a level of about 3°C, whereas when the air diffusers (300) were additionally applied, the temperature change was about 8°C. Accordingly, it was confirmed that the agitation and air diffusion using both the agitators (200) and air diffusers (300) in the case of high heat generation during solid fermentation was more effective in controlling the temperature change of the objects to be fermented.

**[Table 1]**

| | Agitation | Agitation + Diffusion |
|---|---|---|
| Before Application (°C) | 45°C | 47°C |
| After Application (°C) | 42°C | 39°C |
| Amount of Temperature Change (ΔT) | 3°C | 8°C |

As described above, it is possible to perform fermentation at an overall temperature in the range of 37°C to 43°C suitable for *Bacillus,* the seed microorganism, through temperature decrease by agitation. The maintenance of an appropriate temperature for fermentation is regarded as a very important control factor to prevent the adhesion of viscous materials due to the PGA component of *Bacillus* and to maintain the stability of enzyme reactions.

Additionally, in conjunction with this, it was possible to improve the quality of the fermentation product through oxygen supply into the objects to be fermented by the air diffusers (300). One of the most challenging and important factors in the setup of a continuous fermentation device, in particular with regard to the facilities of a continuous fermentation device for aerobic solid fermentation, is an appropriate supply of oxygen to solid objects to be fermented. Insufficient air delivery may cause deterioration in the metabolism of aerobic microorganisms, thus resulting in failure to obtain the desired quality.

In this regard, as in the continuous fermentation device according to the present disclosure, the distribution pipes (320) of the air diffusers (300) were arranged in a transport direction on a conveyor belt (110) to be suitable for the continuous fermentation device using the conveyor belt (110), and the apertures (331) were provided in the distribution pipes (320) so that the apertures (331) are optimized for each size (2 mm to 3 mm) and for each location. Additionally, for optimum oxygen supply as described above, the distribution pipes (320) were arranged in 3 to 8 rows, and the angles of the apertures (331) were adjusted according to the arrangement so that air could be evenly and forcibly ventilated into the objects to be fermented (see FIG. 6).

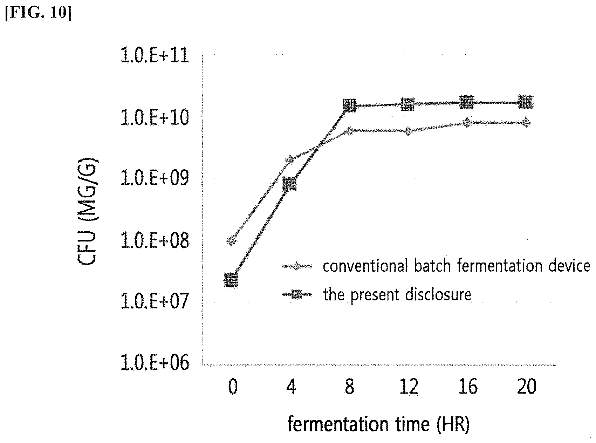

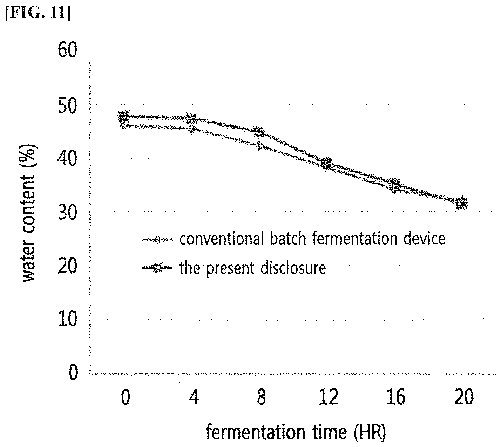

The fermentability and quality of fermentation products were evaluated using the secured air diffusers (300). As shown in FIG. 10, the growth/proliferation of the microorganism was shown to be almost identical to that in the batch fermentation device. As shown in FIG. 11, it was confirmed that the change in water content during solid fermentation was also shown to be at the same rate as in the batch fermentation device. These results show that the heat generated inside the objects to be fermented through aerobic fermentation is appropriately discharged to the outside.

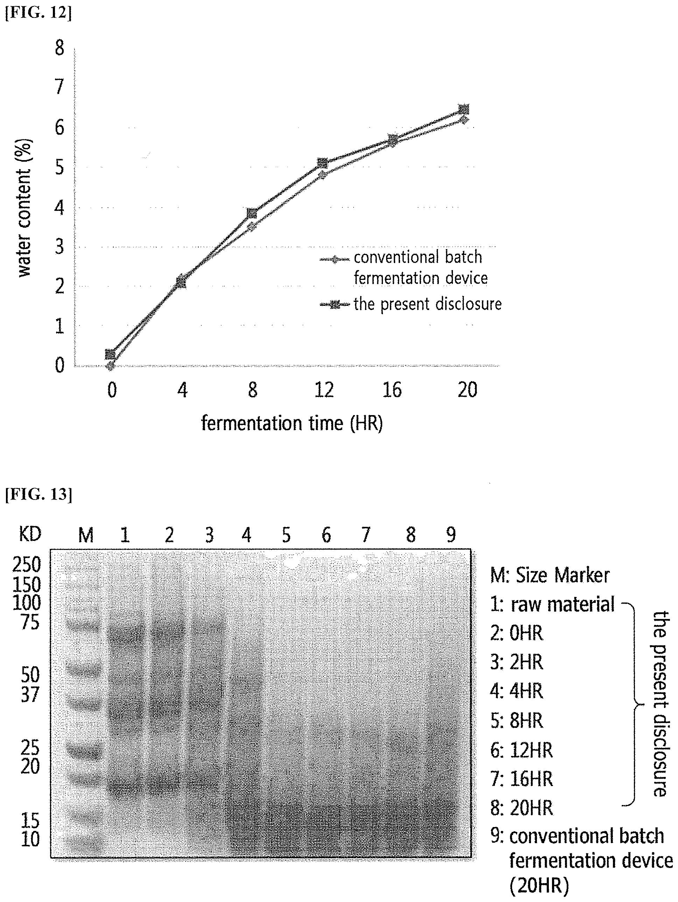

Additionally, as shown in FIG. 12, it was confirmed that the change in the crude protein content rather rapidly increased compared to that in the batch fermentation device. This indicates that, as described above, the effects of enhancing the air permeability of the objects to be fermented through the optimization of the arrangement of the distribution pipes (320) and the arrangement angles of the apertures (331) are exhibited as a change in the overall quality.

Furthermore, the main purpose of the application of the seed microorganism, *Bacillus,* is to induce the peptidation of proteins in soybean meal through enzymatic hydrolysis (the protease in *Bacillus*), and this effect improves feed digestibility, thereby improving feed nutrition efficiency.

As shown in FIG. 13, it was confirmed that when fermentation was performed using the continuous fermentation device according to the present disclosure, protein degradation proceeded well with time. This result showed a protein decomposition pattern similar to that by the batch fermentation device, thus confirming that the utilization of the continuous fermentation device of the present disclosure can secure digestibility equal to or greater than that obtained when the final product quality was utilized.

Then, the activity and KOH solubility of trypsin inhibitor (*i.e.,* a representative inhibitor of digestibility) were measured. As shown in Table 2 below, it was confirmed that the activity of trypsin inhibitor, an anti-nutritional factor, was decreased during the processes of treating raw materials, steaming, and fermentation. Additionally, the KOH solubility was also confirmed to be excellent compared to that prepared by the batch fermentation device, and the comparison of final quality revealed that the quality of the product prepared by the continuous fermentation device of the present disclosure was equal to or higher than that prepared by the batch fermentation device. From these results, it can be concluded that the *Bacillus* fermentation efficiency is superior in the continuous fermentation device according to the present disclosure.

**[Table 2]**

| | Batch Fermentation Device | Present Disclosure (Experiment 1) | Present Disclosure (Experiment 2) | Present Disclosure (Experiment 3) | Present Disclosure (Experiment 4) |
|---|---|---|---|---|---|
| Trypsin Inhibitor (mg/g) | 1.0 or less | 0.72 | 0.67 | 0.62 | 0.73 |
| KOH Solubility | 65 or greater | 77.4 | 82.3 | 81.8 | 83.5 |

Conclusively, oxygen delivery can be more effectively performed by arranging the air diffusers (300) so as to facilitate oxygen supply within the solid objects to be fermented and this will enable the maintenance of optimal fermentation conditions. Additionally, the device of the present disclosure can maximize the solid fermentation of aerobic microorganisms and ensure the quality of the fermentation product.

As described above, the results of the quality of the fermentation product prepared by the device of the present disclosure, which were equal to or greater than that obtained by the conventional batch fermentation device within the same fermentation time, were secured by enhancing the major control factors with regard to solid objects to be fermented, such as temperature of a fermentation product or air feeding into the objects to be fermented (air permeability), and this is a factor that can provide many opportunities for cost reduction by improving productivity through continuous operation and efficient installation space in the progress of industrialization. Additionally, since the amount of load for agitation in the continuous fermentation is less than that in the batch fermentation, the device for agitation can be installed at a lower cost, and the device can be configured to allow ventilation in various places for all of the objects to be fermented, thus being effective for aerobic fermentation. In addition, the device of the present disclosure has an advantage in that it is sufficiently possible to further reduce the investment cost of the facility according to the height of the objects to be fermented.

Additionally, as described above, the guide belt (120) provided on the conveyor belt (110) can prevent falling-off of the objects to be fermented and subsequent contamination thereof. However, there was a concern that surface resistance of the guides (120) may occur during fermentation if fermentation is performed after the guides (120) are provided.

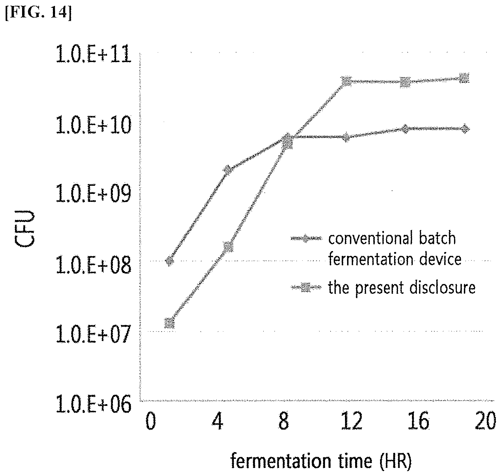

However, as shown in FIG. 14, the guides (120) did not show any surface resistance during this experiment, and it was confirmed that all of the objects to be fermented were sufficient for aerobic fermentation by the air delivered by the distribution pipes (320) described above.

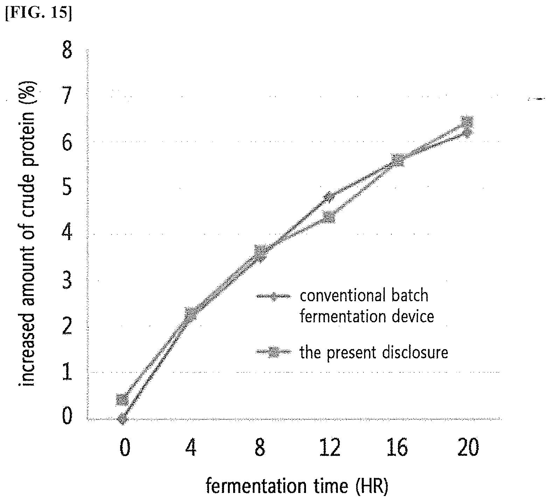

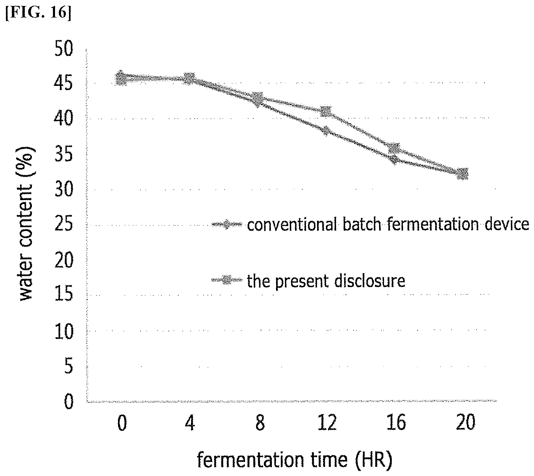

Additionally, as shown in FIGS. 15 and 16, there was also no influence by the guides (120) on the results with regard to the growth of the microorganism, increase of the protein amount, and water content in the final fermentation product of the objects to be fermented. From these results, it is possible to minimize the width of the conveyor belt (110) through the guides (120) and prevent the fermentation product from being spread and falling off after agitation.

Although the preferred embodiments of the present disclosure have been described above, the present disclosure is not limited to the specific embodiments described above. That is, one of ordinary skill in the art to which it belongs will be able to understand that numerous changes and modifications to the present disclosure are possible without departing from the scope of the appended claims.

### (Reference Numerals)

100: Fermentation Module
110: Conveyor Belt
200: Agitator
300: Air Diffuser
400: Pulverizing Unit
500: Air Conditioning Device
600: Air Supply Unit

## Claims

1. A continuous fermentation device comprising a plurality of fermentation modules (100), for being continuously fed with objects to be fermented, and for fermenting the objects while they are passed through the fermentation modules (100), and for continuously discharging the objects after the fermenting, each of the plurality of fermentation modules (100) comprising:
a conveyor belt (110), which transports objects to be fermented that are continuously fed thereinto from one side to the opposite side thereof;
agitators (200), which are located on the conveyor belt (110); **characterised in that** it further comprises
air diffusers (300), which are located on one side of the conveyor belt (110),
wherein the air diffusers (300) each comprise:
a main supply pipe (310), which is located across the top side of the conveyor belt (110) on one side of the conveyor belt (110); and
distribution pipes (320), wherein each distribution pipe extends from one side of the main supply pipe (310) and is located in the longitudinal direction of the top side of the conveyor belt (110), and a plurality of apertures (331) are formed around the periphery thereof,
wherein each of the distribution pipes (320) is provided for injecting air inside the objects to be fermented when the objects to be fermented are transported on the conveyor belt (110).

2. The continuous fermentation device according to claim 1, wherein the plurality of fermentation modules (100) are in a stacked form where one side and the opposite side of each conveyor belt (110) are connected to each other.

3. The continuous fermentation device according to claim 2, wherein the plurality of conveyor belts (110) of the plurality of fermentation modules 100 form a stack of two rows, and each of the plurality of fermentation modules (100) further comprises a belt connection part (150), which connects one side and the opposite side of each of the conveyor belts (110) in each row.

4. The continuous fermentation device according to claim 1, wherein each of the agitators (200) comprises:
a shaft (210), which is located on the conveyor belt (110);
a plurality of support frames (220), wherein each support frame radially extends from one side and the opposite side of the outer circumference of the shaft (210);
connecting frames (230), wherein each connecting frame connects the ends of the support frames (220) located at each of the opposite sides of the shaft (210); and
a plurality of agitating frames (240), wherein each agitating frame connects the connecting frames (230) on one side and the opposite side.

5. The continuous fermentation device according to claim 4, wherein each of the agitators (200) is an agitator (200) for agiting the objects to be fermented, which are transported on the conveyor belt (110) as each of the agitating frames (240) moves, as the shaft (210) rotates.

6. The continuous fermentation device according to claim 4, further comprising agitation wings (250), wherein each agitation wing is located on one side of the outer circumference of the plurality of agitating frames (240) in the longitudinal direction.

7. The continuous fermentation device according to claim 4, wherein the shaft (210) is height-adjustable.

8. The continuous fermentation device according to claim 1, wherein the apertures (331) are formed in at least two rows, the apertures (331) being apertures (331) for supplying therethrough with air the objects to be fermented.

9. The continuous fermentation device according to claim 8, wherein each row formed by the apertures (331) is arranged at a predetermined angle from the center of the distribution pipes (320).

10. The continuous fermentation device according to claim 8, wherein the apertures (331) are arranged in three rows on one side of the outer circumference of the distribution pipes (320), wherein each row formed by the apertures (331) is arranged with an angular difference of from (60) degrees or greater to 90 degrees or less from the center of the distribution pipes (320).

11. The continuous fermentation device according to claim 1, further comprising guides (120), which have a predetermined height and are located in the longitudinal direction on both sides of the conveyor belt (110).

12. The continuous fermentation device according to claim 1, further comprising a conveyor belt (110') for transporting the objects to be fermented, which are fermented and discharged from the conveyor belt (110) to a drying unit.

13. The continuous fermentation device according to claim 1, further comprising:
a plurality of air conditioning devices (500), which are provided on one side of each of the conveyor belt (100) for suction of odors generated during the fermentation process of the objects to be fermented; and
odor-processing units (60) connected to the plurality of air conditioning devices (500),
wherein the odors sucked by the plurality of air conditioning devices (500) are integrated and processed in the odor-processing units (60).

## Patentansprüche

1. Kontinuierliche Fermentationsvorrichtung, umfassend eine Mehrzahl von Fermentationsmodulen (100), um kontinuierlich mit zu fermentierenden Objekten gespeist zu werden, und zum Fermentieren der Objekte, während sie durch die Fermentationsmodule (100) passieren, und zum kontinuierlichen Ausgeben der Objekte nach dem Fermentieren, wobei jedes der Mehrzahl von Fermentationsmodulen (100) umfasst:
ein Förderband (110), das zu fermentierende Objekte, die kontinuierlich dorthinein zugeführt werden, von einer Seite zur gegenüberliegenden Seite davon transportiert;
Mischer (200), die sich auf dem Förderband (110) befinden; **dadurch gekennzeichnet, dass** es ferner Luftverteiler (300) umfasst, die sich auf einer Seite des Förderbandes (110) befinden,
wobei jeder der Luftverteiler (300) umfasst:
ein Hauptversorgungsrohr (310), das sich über der Oberseite des Förderbandes (110) auf einer Seite des Förderbandes (110) befindet; und
Verteilungsrohre (320), wobei sich jedes Verteilungsrohr von einer Seite des Hauptversorgungsrohrs (310) aus erstreckt und sich in Längsrichtung der Oberseite des Förderbandes (110) befindet, und wobei eine Mehrzahl von Öffnungen (331) um dessen Umfang herum ausgebildet sind,
wobei jedes der Verteilungsrohre (320) zum Einblasen von Luft in die zu fermentierenden Objekte vorgesehen ist, wenn die zu fermentierenden Objekte auf dem Förderband (110) transportiert werden.

2. Kontinuierliche Fermentationsvorrichtung nach Anspruch 1, wobei die Mehrzahl von Fermentationsmodulen (100) in einer gestapelten Form vorliegen, wobei eine Seite und die gegenüberliegende Seite eines jeden Förderbands (110) miteinander verbunden sind.

3. Kontinuierliche Fermentationsvorrichtung nach Anspruch 2, wobei die Mehrzahl von Förderbändern (110) der Mehrzahl von Fermentationsmodulen 100 einen Stapel von zwei Reihen ausbilden, und jedes der Mehrzahl von Fermentationsmodulen (100) ferner ein Bandverbindungsteil (150) umfasst, das eine Seite und die gegenüberliegende Seite eines jeden der Förderbänder (110) in jeder Reihe verbindet.

4. Kontinuierliche Fermentationsvorrichtung nach Anspruch 1, wobei jeder der Mischer (200) umfasst:
eine Welle (210), die sich auf dem Förderband (110) befindet;
eine Mehrzahl von Stützrahmen (220), wobei sich jeder Stützrahmen radial von einer Seite und der gegenüberliegenden Seite des Außenumfangs der Welle (210) aus erstreckt;
Verbindungsrahmen (230), wobei jeder Verbindungsrahmen die Enden der Stützrahmen (220) verbindet, die sich an jeder der einander gegenüberliegenden Seiten der Welle (210) befinden; und
eine Mehrzahl von Mischrahmen (240), wobei jeder Mischrahmen die Verbindungsrahmen (230) auf einer Seite und der gegenüberliegenden Seite verbindet.

5. Kontinuierliche Fermentationsvorrichtung nach Anspruch 4, wobei jeder der Mischer (200) ein Mischer (200) zum Bewegen der zu fermentierenden Objekte ist, die auf dem Förderband (110) transportiert werden, wenn sich jeder der Mischrahmen (240) bewegt und sich die Welle (210) dreht.

6. Kontinuierliche Fermentationsvorrichtung nach Anspruch 4, ferner umfassend Mischflügel (250), wobei jeder Mischflügel auf einer Seite des Außenumfangs der Mehrzahl von Mischrahmen (240) in Längsrichtung angeordnet ist.

7. Kontinuierliche Fermentationsvorrichtung nach Anspruch 4, wobei die Welle (210) höhenverstellbar ist.

8. Kontinuierliche Fermentationsvorrichtung nach Anspruch 1, wobei die Öffnungen (331) in mindestens zwei Reihen ausgebildet sind, wobei die Öffnungen (331) Öffnungen (331) sind, um durch diese hindurch die zu fermentierenden Objekte mit Luft zu versorgen.

9. Kontinuierliche Fermentationsvorrichtung nach Anspruch 8, wobei jede durch die Öffnungen (331) ausgebildete Reihe in einem vorgegebenen Winkel von der Mitte der Verteilerrohre (320) angeordnet ist.

10. Kontinuierliche Fermentationsvorrichtung nach Anspruch 8, wobei die Öffnungen (331) in drei Reihen auf einer Seite des Außenumfangs der Verteilungsrohre (320) angeordnet sind, wobei jede durch die Öffnungen (331) ausgebildete Reihe mit einer Winkeldifferenz von mindestens 60 Grad bis höchstens 90 Grad von der Mitte der Verteilungsrohre (320) angeordnet ist.

11. Kontinuierliche Fermentationsvorrichtung nach Anspruch 1, ferner umfassend Führungen (120), die eine vorbestimmte Höhe aufweisen und sich in Längsrichtung auf beiden Seiten des Förderbandes (110) befinden.

12. Kontinuierliche Fermentationsvorrichtung nach Anspruch 1, ferner umfassend ein Förderband (110') für den Transport von zu fermentierenden Objekten, die fermentiert und von dem Förderband (110) in eine Trocknungseinheit ausgegeben werden.

13. Kontinuierliche Fermentationsvorrichtung nach Anspruch 1, ferner umfassend:
ine Mehrzahl von Klimaanlagen (500), die auf einer Seite eines jeden der Förderbänder (100) zum Absaugen von Gerüchen vorgesehen sind, die während des Fermentationsprozesses der zu fermentierenden Objekte erzeugt werden; und
Geruchsverarbeitungseinheiten (60), die mit der Mehrzahl von Klimaanlagen (500) verbunden sind,
wobei die durch die Mehrzahl von Klimaanlagen (500) angesaugten Gerüche in die Geruchsverarbeitungseinheiten (60) eingeführt und dort verarbeitet werden.

## Revendications

1. Dispositif de fermentation continue comprenant plusieurs modules de fermentation (100), destinés à être alimentés en continu en objets à fermenter, à fermenter les objets pendant qu'ils traversent les modules de fermentation (100) et à décharger les objets en continu après qu'ils ont fermenté, chacun des modules de fermentation (100) comprenant :
une bande transporteuse (110) qui transporte d'un côté à son côté opposé les objets à fermenter qui y sont introduits en continu et
des agitateurs (200) situés sur la bande transporteuse (110), **caractérisé en ce qu'**il comprend en outre :
des diffuseurs d'air (300) situés sur un côté de la bande transporteuse (110),
chacun des diffuseurs d'air (300) comprenant :
un conduit principal d'admission (310) situé au-dessus du côté supérieur de la bande transporteuse (110), sur un côté de la bande transporteuse (110) et
des conduits de répartition (320), chaque conduit de répartition partant d'un côté du conduit principal d'admission (310) et étant disposé dans le sens de la longueur du côté supérieur de la bande transporteuse (110), plusieurs orifices (331) étant formés à sa périphérie,
chacun des conduits de répartition (320) étant configuré pour injecter de l'air à l'intérieur des objets à fermenter pendant que les objets à fermenter sont transportés sur la bande transporteuse (110).

2. Dispositif de fermentation continue selon la revendication 1, dans lequel les différents modules de fermentation (100) sont disposés sous forme empilée avec un côté et le côté opposé de chaque bande transporteuse (110) reliés l'un à l'autre.

3. Dispositif de fermentation continue selon la revendication 2, dans lequel les différentes bandes transporteuses (110) des différents modules de fermentation (100) forment un empilement en deux rangées, chacun des différents modules de fermentation (100) comprenant en outre une pièce de raccordement de courroie (150) qui raccorde un côté et le côté opposé de chacune des bandes transporteuses (110) de chaque rangée.

4. Dispositif de fermentation continue selon la revendication 1, dans lequel chacun des agitateurs (200) comprend :
un arbre (210) situé sur la bande transporteuse (110),
plusieurs bâtis de support (220), chaque bâti de support débordant radialement d'un côté et du côté opposé de la circonférence extérieure de l'arbre (210),
plusieurs bâtis de raccordement (230), chaque bâti de raccordement reliant les extrémités des bâtis de support (220) situés sur chacun des côtés opposés de l'arbre (210) et
plusieurs bâtis d'agitation (240), chaque bâti d'agitation reliant les bâtis de raccordement (230) d'un côté et du côté opposé.

5. Dispositif de fermentation continue selon la revendication 4, dans lequel chacun des agitateurs (200) est un agitateur (200) qui sert à agiter les objets à fermenter transportés sur la bande transporteuse (110) lorsque chacun des bâtis d'agitation (240) se meut pendant que l'arbre (210) tourne.

6. Dispositif de fermentation continue selon la revendication 4, comprenant en outre des ailettes d'agitation (250), chaque ailette d'agitation (250) étant située dans le sens de la longueur sur un côté de la circonférence extérieure des différents bâtis d'agitation (240).

7. Dispositif de fermentation continue selon la revendication 4, dans lequel la hauteur de l'arbre (210) est ajustable.

8. Dispositif de fermentation continue selon la revendication 1, dans lequel les orifices (331) sont formés en au moins deux rangées, les orifices (331) étant des orifices (331) qui alimentent en air les objets à fermenter.

9. Dispositif de fermentation continue selon la revendication 8, dans lequel chaque rangée formée par les orifices (331) est disposée à un angle prédéterminé par rapport au centre des conduits de répartition (320).

10. Dispositif de fermentation continue selon la revendication 8, dans lequel les orifices (331) sont agencés en trois rangées sur un côté de la circonférence extérieure des conduits de répartition (320), chaque rangée formée par les orifices (331) étant agencée à un écart angulaire de 60 degrés ou plus à 90 degrés ou moins par rapport au centre des conduits de répartition (320).

11. Dispositif de fermentation continue selon la revendication 1, comprenant en outre des guides (120) de hauteur prédéterminée et disposés dans le sens de la longueur sur les deux côtés de la bande transporteuse (110) .

12. Dispositif de fermentation continue selon la revendication 1, comprenant en outre une bande transporteuse (110') qui sert à transporter vers une unité de séchage les objets à fermenter après que ces derniers ont fermenté et sont déchargés de la bande transporteuse (110).

13. Dispositif de fermentation continue selon la revendication 1, comprenant en outre :
plusieurs dispositifs de traitement d'air (500) prévus sur un côté de chacune des bandes transporteuses (110) pour aspirer des odeurs dégagées pendant le processus de fermentation des objets à fermenter et
des unités (60) de traitement des odeurs raccordées aux dispositifs de traitement d'air (500), les odeurs aspirées par les dispositifs de traitement d'air (500) étant introduites dans les unités (60) de traitement des odeurs pour y être traitées.
